# EUROPÄISCHE PATENTSCHRIFT

(11) **EP 0 643 573 B1**
(45) Veröffentlichungstag und Bekanntmachung des Hinweises auf die Patenterteilung: **11.09.1996**
(21) Anmeldenummer: 93912824.5
(22) Anmeldetag: 28.05.1993
(51) Int. Cl.: A61K 7/11

(54) **HAARPFLEGEMITTEL**
HAIR CARE PRODUCT
PRODUIT DE SOINS CAPILLAIRES

(30) Priorität: 05.06.1992 DE 4218583
(43) Veröffentlichungstag der Anmeldung: 22.03.1995
(73) Patentinhaber: Henkel Kommanditgesellschaft auf Aktien, 40191 Düsseldorf (DE)
(72) Erfinder: WÜLKNITZ, Peter, D-4018 Langenfeld (DE); HOFMANN, Hans-Peter, D-4000 Düsseldorf (DE)
(86) Internationale Anmeldenummer: EP9301351
(87) Internationale Veröffentlichungsnummer: WO9325180

(56) Entgegenhaltungen:
- EP-A- 0 208 951
- EP-A- 0 331 930
- WO-A-91/08730
- FR-A- 2 222 995
- US-A- 5 164 177

## Beschreibung

Gegenstand der Erfindung sind Zubereitungen zum Festigen von Haaren auf Basis einer wäßrigen oder wäßrig-alkoholischen Lösung mit einer speziellen Treibmittelmischung.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung bestimmter Haarfestlegemittel aufbauen bzw. für einen längeren Zeitraum aufrechterhalten lassen. Solche Haarfestlegemittel werden üblicherweise in Form von Haarsprays bereitgestellt und enthalten als zwingende Komponenten in der Regel folgende Bestandteile:
- Lösungsmittel, üblicherweise ein niederer Alkohol,
- Filmbildner
- Treibmittel.

Obwohl diese Rezepturen im Vergleich zu anderen Kosmetika und Körperreinigungsmitteln auf den ersten Blick einfach aufgebaut scheinen, können sie doch in einer Reihe von Punkten noch nicht befriedigen.

Wurden früher Fluorchlorkohlenwasserstoffe als ideale, inerte Treibmittel angesehen, so führte die aktuelle Ozon-Diskussion zu einem weitestgehenden Ersatz dieser ökologisch bedenklichen Treibmittel. Heute werden in der Regel als Treibmittel niedere Alkane wie Propan/Butan oder Dimethylether verwendet. Deren Verwendung erfordert entweder einen insbesondere beim Abfüllen deutlich größeren technischen Aufwand oder ist mit anderen Nachteilen, z.B. leichter Brennbarkeit, behaftet.

Dies führte einerseits zu einer Renaissance der Pumpsprays, bei denen treibmittelfreie Lösungen mittels einer mechanischen Vorrichtung versprüht werden. Hier bedarf es nicht unwesentlicher technischer Anstrengungen, um ein Sprühverhalten zu erhalten, das dem der FCKW-haltigen Sprays nahekommt.

Zum anderen wird ein Versprühen der Formulierungen mit bekannten, wohlfeilen Gasen wie CO₂, Stickstoff N₂O oder Luft angestrebt. Die Verwendung einzelner dieser Treibgase oder Mischungen dieser Treibgase in Haarbehandlungsmitteln sind beispielsweise aus den Druckschriften WO-A-9108730, US-A-5164177 und EP-A-0 208 951 bekannt. EP-A-0 331 930 offenbart die Kombination dieser Treibgase mit weiteren Treibmitteln.

Bei der Verwendung von Gasen, die in den zu versprühenden Formulierungen praktisch unlöslich sind, ist aber mit der zunehmenden Entleerung des Behälters unweigerlich ein starker Druckabfall verbunden. Ein Druckabfall auf weniger als die Hälfte des Ausgangsdruckes, der aus technischen Gründen kaum über ca. 10 bar gesteigert werden kann, führt zu massiven Veränderungen des Sprühverhaltens. Diese Veränderungen sind gerade bei höherviskosen Lösungen, wie sie Haarsprayformulierungen darstellen, nicht mehr akzeptabel.

Verwendet man dagegen Gase, die sich in der zu versprühenden Lösung in größeren Mengen lösen, so sinkt der Doseninnendruck bis zum vollständigen Entleeren weit weniger ab, da laufend Gas aus der Lösung "nachgeliefert" wird. Solche Mittel sind beispielsweise aus der Druckschrift FR-A-2222995 bekannt. Nachteile dieser Gase, deren bekanntester Vertreter das CO₂ ist, sind aber mögliche chemische Reaktionen mit anderen Rezepturbestandteilen sowie eine negative Beeinflussung des Sprühverhaltens in Hinblick auf Nachsprühen, Nachlaufen aus der Dose etc.

Es wurde nun gefunden, daß sich haarfestigende Zubereitungen, deren Viskosität nicht zu hoch ist, mit einer Mischung aus löslichen und unlöslichen Treibgasen überraschend gut versprühen lassen. Die Zuhereitungen verkleben nicht und zeichnen sich durch schnelle Trockenzeiten auf dem Haar sowie eine gute Festigung der Frisur aus.

Gegenstand der Erfindung sind daher Zubereitungen zum Festigen von Haaren auf Basis einer alkoholischen oder wäßrig-alkoholischen Lösung, enthaltend Filmbildner, Lösungsmittel, Treibmittel und übliche kosmetische Bestandteile, gekennzeichnet durch
a) eine Treibmittelmischung, bestehend aus einem gasförmigem Treibmittel A, das in der Lösung unlöslich oder zu weniger als 5 Vol-% bei 20 °C und 1 bar Partialdruck löslich ist, und einem gasförmigen Treibmittel B, das in der Lösung bei 20 °C und 1 bar Partialdruck zu mindestens 40 Vol-% löslich ist, im Massenverhältnis 3:1 bis 1:10 und
b) eine dynamische Viskosität der Lösung zwischen 1,5 und 10 mPas bei Raumtemperatur.

Als Treibmittel A können insbesondere Luft, Stickstoff und Edelgase verwendet werden. Die Verwendung von Luft oder Stickstoff ist bevorzugt. Die Treibmittel A sind in den erfindungsgemäßen Zubereitungen bevorzugt in Mengen von 0,1 bis 1,5 Gew.-%, bezogen auf die gesamte Zubereitung, enthalten. Mengen von 0,2 bis 1,0 Gew.-% sind besonders bevorzugt.

Bevorzugte Treibmittel B sind Kohlendioxid und Distickstoffoxid. Die Treibmittel B sind in den erfindungsgemäßen Zubereitungen bevorzugt in Mengen von 0,2 bis 8 Gew.-%, bezogen auf die gesamte Zubereitung, enthalten. Mengen von 0,5 bis 5 Gew.-% sind besonders bevorzugt. Im Falle von Kohlendioxid als Treibmittel kann die gewünschte Menge sowohl aus Druckgasbehältern abgefüllt als auch durch Zugabe der entsprechenden Menge Trockeneis zur Verfügung gestellt werden. Gleichfalls ist es möglich, das Kohlendioxid durch Einwirkung von organischen Säuren auf Salze der Kohlensäure wie beispielsweise Calcium- und Natriumcarbonat erst in dem bereits verschlossenen, gebrauchsfertigen Behälter zu entwickeln. Das Einbringen von Kohlendioxid als Gas aus Druckgasbehältern ist üblicherweise bevorzugt.

Das Verhältnis der Massen von Treibmittel A und Treibmittel B liegt erfindungsgemäß zwischen 3:1 und 1:10. Verhältnisse von 1:1 bis 1:6 sind bevorzugt.

Ein wesentlicher Parameter der erfindungsgemäßen Zubereitungen ist die Viskosität der Lösung. Diese muß zwischen 1,5 und 10 mPas bei Raumtemperatur, d.h. bei ca. 20 °C, liegen, damit die Zubereitungen beim Versprühen die gewünschten positiven Eigenschaften aufweisen. Zur Bestimmung der Viskosität kann ein Ubbelohde-Viskosimeter Typ 501 unter Verwendung einer Kapillare vom Typ Ic mit einem Durchmesser von 0,84 mm eingesetzt werden.

Zubereitungen mit Viskositäten zwischen 1,5 und 8 mPas zeigen besonders gute Eigenschaften.

Als Hauptkomponente enthalten die Zubereitungen einen niederen Alkohol. Bevorzugte Alkohole sind Ethanol, n-Propanol und Isopropanol. Ethanol ist besonders bevorzugt. Der Alkohol ist üblicherweise in Mengen von 40 bis 95 Gew.-%, bezogen auf die gesamte Zubereitung, enthalten. Mengen von 50 bis 95 Gew.-%, insbesondere Mengen über 75 Gew.-%, sind bevorzugt.

Die erfindungsgemäßen Zubereitungen können Alkohol als einziges Lösungsmittel enthalten. Es können aber auch weitere Lösungsmittel in Mengen bis zu 60 Gew.-%, bezogen auf die gesamte Zubereitung, enthalten sein. Geeignete Lösungsmittel sind zum Beispiel Wasser, lineare und verzweigte Alkane mit 5 bis 7 C-Atomen und Ketone mit 3 bis 6 C-Atomen. Wasser, n-Pentan, n-Heptan, Aceton und 3-Methyl-3-methoxy-butanol sind bevorzugte Lösungsmittel. Wasser und n-Heptan, jeweils in Mengen bis zu etwa 30 Gew.-%, insbesondere bis zu 20 Gew. -%, sind besonders bevorzugt.

Durch geeignete Wahl der Lösungsmittel ist es möglich, die Zubereitungen als klare Lösungen zu formulieren. Solche klare Lösungen werden vom Verbraucher üblicherweise bereits aus ästhetischen Gründen bevorzugt. Sofern die erfindungsgemäßen Mittel daher in durchsichtigen Behältern formuliert werden, sind daher Zubereitungen in Form klarer Lösungen bevorzugt.

Weiterhin enthalten die erfindungsgemäßen Haarfestlegemittel als zwingenden Bestandteil 0,5 - 10 Gew.-% eines Filmbildners. Als Filmbildner können die bekannten natürlichen Harze, Kunstharze und synthetischen Polymeren verwendet werden, die im gewählten Lösungsmittel löslich sind und nach dem Verdunsten des Lösungsmittels auf dem Haar einen Überzug hinterlassen.

Erfindungsgemäß bevorzugte Filmbildner sind nichtionogene, amphotere, zwitterionische und anionische Polymere.

Geeignete nichtionogene Polymere sind beispielsweise:
- Vinylpyrrolidon/Vinylacetat-Copolymere, wie sie beispielsweise unter dem Warenzeichen Luviskol^{R} (BASF) vertrieben werden. Luviskol^{R} VA 64 Luviskol^{R} VA 73 und Luviskol^{R} VA 37 sind bevorzugte nichtionogene Polymere; Luviskol^{R} VA 37 ist besonders bevorzugt.
- Vinylpyrrolidon/Dimethylaminoethylmethacrylat/Vinylcaprolactam-Terpolymere, wie sie beispielsweise unter der Bezeichnung Copolymer VC-713 (GAF) erhältlich sind.

Geeignete amphotere Polymere sind beispielsweise die unter den Bezeichnungen Amphomer^{R} und Amphomer^{R} LV-71 (Delft National) erhältlichen Octylacrylamid/Methylmethacrylat/tert. Butylaminoethylmethacrylat/2-Hydroxypropylmethacrylat-Copolymere.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäure-Copolymerisate und deren Alkali- und Ammoniumsalze sind besonders bevorzugte zwitterionische Polymere. Weiterhin geeignete zwitterionische Polymere sind Methacroylethylbetain/Methacrylat-Copolymere, die unter der Bezeichnung Amersette^{R} (Amerchol) im Handel erhältlich sind.

Erfindungsgemäß geeignete anionische Polymere sind u. a.:
- Vinylacetat/Crotonsäure-Copolymere, wie sie beispielsweise unter den Bezeichnungen Resyn^{R} (National Starch), Luviset^{R} (BASF) und Gafset^{R} (GAF) im Handel sind. Luviset^{R} CA-66 ist ein besonders bevorzugtes anionisches Polymeres.
- Vinylpyrrolidon/Vinylacrylat-Copolymere, erhältlich beispielsweise unter dem Warenzeichen Luviflex^{R} (BASF). Ein bevorzugtes Polymer ist das unter der Bezeichnung Luviflex^{R} VBM-35 (BASF) erhältliche Vinylpyrrolidon/Acrylat-Terpolymere.
- Vinylacetat/Butylmaleat/Isobornylacrylat-Copolymere, wie sie unter dem Warenzeichen Advantage^{R} (GAF) erhältlich sind. Advantage^{R} CP ist ein bevorzugtes Polymeres.
- Methylvinylether/Maleinsäureanhydrid-Copolymere und deren Ester, wie sie beispielsweise unter der Bezeichnung Gantrez^{R} (GAF) erhältlich sind. Gantrez^{R} ES 225 ist ein bevorzugtes anionisches Polymer.
- Acrylsäure/Ethylacrylat/N-tert.Butylacrylamid-Terpolymere, die beispielsweise unter der Bezeichnung Ultrahold^{R}8 (BASF) vertrieben werden.

Nichtionogene Polymere wie Luviskol VA 37 und anionische Polymere wie Luviflex VBM 35 und Ultrahold^{R}8 sind besonders bevorzugt.

Mittel mit einem Gehalt an Filmbildnern von 1 - 7, insbesondere von 3 bis 7, Gew.-%, sind erfindungsgemäß bevorzugt.

Weiterhin können die erfindungsgemäßen Haarfestlegemittel alle für solche Mittel bekannten kosmetischen Inhaltsstoffe enthalten. Diese weiteren Inhaltsstoffe sind üblicherweise nur in untergeordneten Mengen, d.h. in der Regel in Mengen von bis zu einigen Gew.-%, enthalten.

Solche üblichen Bestandteile sind beispielsweise:
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe,
- Wirkstoffe wie Panthenol, Allantoin, Pyrrolidoncarbonsäuren, Pflanzenextrakte und Vitamine,
- Lichtschutzmittel,
- Konsistenzgeber wie Anlagerungsprodukte von 5 bis 60 Mol Ethylenoxid an Rizinusöl und gehärtetes Rizinusöl,
- Kationische Polymere wie beispielsweise quaternisierte Celluloseether, Polysiloxane mit quaternären Gruppen, Acrylamid-Dimethyldiallylammoniumchlorid-Copolymere sowie mit Diethylsulfat quaternierte Dimethylaminomethacrylat-Vinylpyrrolidon-Copolymere und
- Silikonöle.

Die erfindungsgemäßen Haarfestlegemittel können weiterhin gewünschtenfalls geringe Mengen an Konservierungsmitteln enthalten. Es wurde aber festgestellt, daß die erfindungsgemäßen Zubereitungen in aller Regel auch ohne weitere Konservierungsstoffe eine ausreichende Stabilität gegen Keimbefall aufweisen. Konservierungsmittelfreie Formulierungen sind daher im Rahmen der erfindungsgemäßen Lehre bevorzugt.

Die erfindungsgemäßen Zubereitungen sind mit allen üblichen, für Haarsprays geeigneten Behältern appliziert worden. Durch Wahl spezieller Ventile kann dem Treibgut allerdings noch ein geringer Anteil des Druckgases zugemischt werden. Dadurch erhält man eine etwas kleinere Tröpfchengröße und eine kräftigere Sprühcharakteristik. Die erfindungsgemäßen Zubereitungen werden daher bevorzugt mittels solcher Ventile appliziert.

Gegenstand der Erfindung ist ebenfalls ein Verfahren zur Behandlung, insbesondere zum Festigen von Haaren, dadurch gekennzeichnet, daß eine Zubereitung nach einem der Ansprüche 1 bis 9 verwendet wird.

Die folgenden Beispiele sollen den Gegenstand der Erfindung weiter erläutern.

### Beispiele

Es wurden folgende Formulierungen hergestellt (Angaben in Gewichtsteilen):

| Komponente | Beispiel 1 | Vergleich 1 | Vergleich 2 |
|---|---|---|---|
| Ethanol abs. | 80,0 | 80,0 | 80,0 |
| Wasser | 14,8 | 14,8 | 14,8 |
| Luviset^{R}VBM 35¹ | 5,0 | 5,0 | 5,0 |
| Parfümöl | 0,2 | 0,2 | 0,2 |
| Stickstoff | 1,0 | 1,1 | - |
| Kohlendioxid | 0,9 | - | 5,9 |
| Dosendruck befüllt [bar] | 9 | 9 | 9 |

| | | | |
|---|---|---|---|
| ¹ Vinylpyrrolidon/Acrylat-Terpolymeres (50 % in Ethanol) (BASF). | | | |

Beim Versprühen mit einer handelsüblichen Spraydose (Aluminium-Dose mit einem Regulator-Sprühkopf (Novospray) und einem Standard-Ventil der Fa. DPV) wurden folgende Ergebnisse erzielt:

| Komponente | Beispiel 1 | Vergleich 1 | Vergleich 2 |
|---|---|---|---|
| mittlere Tröpfchengröße [Mikrometer] | | | |
| Standard-Ventil | 72 | 74 | 72 |
| Misch-Ventil | 62 | - | 60 |
| Düsenverklebung | nein | ja | nein |
| Nachlaufen, Zischen | nein | nein | ja |
| Sprühcharakteristik | ausgeglichen | schwach | scharf |
| Tropfenverlauf auf dem Haar | gut | befriedigend | gut |
| Festigungswirkung | gut | befriedigend | gut |

### Beispiel 2

| Komponente | Gewichtsteile |
|---|---|
| Ethanol abs. | 79,75 |
| Wasser | 5,00 |
| n-Heptan | 10,00 |
| Luviskol^{R}VA 37² | 5,00 |
| Parfümöl | 0,25 |
| Stickstoff | 0,60 |
| Kohlendioxid | 3,00 |
| Dosendruck befüllt [bar] | 9 |

| | |
|---|---|
| ¹ Vinylpyrrolidon/Vinylacetat(30:70)-Copolymeres (BASF) | |

### Beispiel 3

| Komponente | Gewichtsteile |
|---|---|
| Ethanol abs. | 84,75 |
| n-Heptan | 10,00 |
| Luviskol^{R}VA 37 | 5,00 |
| Parfümöl | 0,25 |
| Luft | 0,70 |
| Kohlendioxid | 3,00 |

### Beispiel 4

| Komponente | Gewichtsteile |
|---|---|
| Ethanol abs. | 79,35 |
| Wasser | 5,00 |
| n-Heptan | 10,00 |
| Ultrahold8³ | 5,00 |
| Parfümöl | 0,20 |
| 2-Amino-2-methyl-1-propanol | 0,45 |
| Stickstoff | 0,60 |
| Distickstoffoxid | 2,90 |

| | |
|---|---|
| ³ Acrylsäure/Ethylacrylat/N-tert.Butylacrylamid-Terpolymeres (BASF) | |

### Beispiel 5

| Komponente | Gewichtsteile |
|---|---|
| Ethanol abs. | 80,20 |
| Wasser | 5,00 |
| n-Heptan | 10,00 |
| Luviskol^{R}VA 37 | 4,00 |
| Parfümöl | 0,25 |
| 2-Amino-2-methyl-1-propanol | 0,45 |
| Paridol^{R}M⁴ | 0,1 |
| Stickstoff | 0,60 |
| Kohlendioxid | 3,00 |

| | |
|---|---|
| ⁴ 4-Hydroxybenzoesäuremethylester (NAARDEN) | |

## Patentansprüche

1. Zubereitung zum Festigen von Haaren auf Basis einer alkoholischen oder wäßrig-alkoholischen Lösung, enthaltend Filmbildner, Lösungsmittel, Treibmittel und übliche kosmetische Bestandteile, gekennzeichnet durch
a) eine Treibmittelmischung, bestehend aus einem gasförmigem Treibmittel A, das in der Lösung unlöslich oder zu weniger als 5 Vol-% bei 20 °C und 1 bar Partialdruck löslich ist, und einem gasförmigen Treibmittel B, das in der Lösung bei 20 °C und 1 bar Partialdruck zu mindestens 40 Vol-% löslich ist, im Massenverhältnis 3:1 bis 1:10 und
b) eine dynamische Viskosität der Lösung zwischen 1,5 und 10 mPas bei 20°C.

2. Zubereitung nach Anspruch 1, dadurch gekennzeichnet, daß das Treibmittel A Luft oder Stickstoff ist.

3. Zubereitung nach Anspruch 1 oder 2, dadurch gekennzeichnet, daß das Treibmittel A in einer Menge von 0,1 bis 1,5 Gew.-%, bezogen auf die gesamte Zubereitung, enthalten ist.

4. Zubereitung nach einem der Ansprüche 1 bis 3, dadurch gekennzeichnet, daß das Treibmittel B Kohlendioxid oder Distickstoffoxid ist.

5. Zubereitung nach einem der Ansprüche 1 bis 4, dadurch gekennzeichnet, daß das Treibmittel B in einer Menge von 0,2 bis 8 Gew.-%, bezogen auf die gesamte Zubereitung, enthalten ist.

6. Zubereitung nach einem der Ansprüche 1 bis 5, dadurch gekennzeichnet, daß die dynamische Viskosität der Lösung bei Raumtemperatur zwischen 1,5 und 8 mPas liegt.

7. Zubereitung nach einem der Ansprüche 1 bis 6, dadurch gekennzeichnet, daß als Filmbildner (b) ein nichtionogenes oder anionisches Polymeres enthalten ist.

8. Zubereitung nach einem der Ansprüche 1 bis 7, dadurch gekennzeichnet, daß der Filmbildner in einer Menge von 1 - 7, insbesondere von 3 bis 7, Gew.-% enthalten ist.

9. Zubereitung nach einem der Ansprüche 1 bis 7, dadurch gekennzeichnet, daß es frei von Konservierungsmitteln ist.

10. Verfahren zur Behandlung von Haaren, dadurch gekennzeichnet, daß eine Zubereitung nach einem der Ansprüche 1 bis 9 verwendet wird.

## Claims

1. A hair-setting preparation based on an alcoholic or aqueous/alcoholic solution containing film formers, solvents, propellants and typical cosmetic components, characterized by a) a propellant mixture consisting of a gaseous propellant A, which is insoluble in the solution or of which less than 5% by volume is soluble therein at 20°C/1 bar partial pressure, and a gaseous propellant B, of which at least 40% by volume is soluble in the solution at 20°C/1 bar partial pressure, in a ratio by weight of 3:1 to 1:10 and b) a dynamic viscosity of the solution of 1.5 to 10 mPas at 20°C.

2. A preparation as claimed in claim 1, characterized in that the propellant A is air or nitrogen.

3. A preparation as claimed in claim 1 or 2, characterized in that the propellant A is present in a quantity of 0.1 to 1.5% by weight, based on the preparation as a whole.

4. A preparation as claimed in any of claims 1 to 3, characterized in that the propellant B is carbon dioxide or dinitrogen oxide.

5. A preparation as claimed in any of claims 1 to 4, characterized in that the propellant B is present in a quantity of 0.2 to 8% by weight, based on the preparation as a whole.

6. A preparation as claimed in any of claims 1 to 5, characterized in that the dynamic viscosity of the solution at room temperature is between 1.5 and 8 mPas.

7. A preparation as claimed in any of claims 1 to 6, characterized in that a nonionic or anionic polymer is present as the film former (b).

8. A preparation as claimed in any of claims 1 to 7, characterized in that the film former is present in a quantity of 1 to 7% by weight and more particularly 3 to 7% by weight.

9. A preparation as claimed in any of claims 1 to 7, characterized in that it is free from preservatives.

10. A process for the treatment of hair, characterized in that the preparation claimed in any of claims 1 to 9 is used.

## Revendications

1. Préparation pour la fixation des cheveux à base d'une solution alcoolique ou alcoolique aqueuse, contenant des produits filmogènes, des solvants, des agents propulseurs et d'autres composants cosmétiques,
caractérisée par
a) un mélange d'agents propulseur, composé d'un gaz propulseur A, qui est insoluble dans la solution ou est soluble à moins de 5 % en volume à 20°C et 1 bar de pression partielle et d'un agent propulseur B gazeux, qui est soluble dans la solution à 20°C et 1 bar de pression partielle au moins à 40 % en volume, dans un rapport de masses 3:1 à 1:10 et,
b) une viscosité dynamique de la solution comprise entre 1,5 et 10 mPas à 20°C.

2. Préparation selon la revendication 1,
caractérisée en ce que
l'agent propulseur A est de l'air ou de l'azote.

3. Préparation selon les revendications 1 ou 2,
caractérisée en ce que
l'agent propulseur A est contenu en une quantité de 0,1 à 1,5 % en poids, par rapport à la préparation totale.

4. Préparation selon une des revendications 1 à 3,
caractérisée en ce que
l'agent propulseur B est du dioxyde de carbone ou du protoxyde d'azote.

5. Préparation selon une des revendications 1 à 4,
caractérisée en ce que
l'agent propulseur B est contenu en une quantité de 0,2 à 8 % en poids, par rapport à la préparation totale.

6. Préparation selon une des revendications 1 à 5,
caractérisée en ce que
la viscosité dynamique de la solution se situe à température ambiance entre 1,5 et 8 mPas.

7. Préparation selon une des revendications 1 à 6,
caractérisée en ce que
comme agent filmogène (b) est contenu un polymère non ionogène ou anionique.

8. Préparation selon une des revendications 1 à 7,
caractérisée en ce que
l'agent filmogène est contenu en une quantité de 1-7, en particulier de 3 à 7 % en poids.

9. Préparation selon une des revendications 1 à 7,
caractérisée en ce que
elle est privée d'agents de conservation.

10. Procédé pour le traitement des cheveux,
caractérisé en ce que
on utilise une préparation selon une des revendications 1 à 9.
